# EUROPEAN PATENT APPLICATION

(11) **EP 0 909 568 A2**
(43) Date of publication of application: **21.04.1999**
(21) Application number: 98307891.6
(22) Date of filing: 29.09.1998
(51) Int. Cl.: A61N 1/30

(54) **Methods for implementing current delivery profiles used in an iontophoretic system**

(30) Priority: 29.09.1997 US 939771
(71) Applicant: Becton, Dickinson and Company, Franklin Lakes, New Jersey 07417-1880 (US)
(72) Inventor: Walter, Steven D., Vernon, NJ 07462 (US); Garde, Kenneth E., New Windsor, NY 12553 (US); Flower, Ronald J., Lawrenceville, GA 30045 (US)
(74) Representative: Ruffles, Graham Keith

(57) **Abstract**

An iontophoretic system is provided, including a patch and a controller. The patch includes a plurality of electrodes electrically connectable to the controller and through which current is applied by the controller to the skin. The controller includes at least one current delivery profile, an input device, a control circuitry, and circuitry for generating the patch current. The current delivery profile is an element that can be changed independently of the other components of the controller. The control circuitry selects a current delivery profile using an input from the input device, and causes the current generating circuitry to generate and apply current to the patch electrodes in accordance with the read current delivery profile.

## Description

### FIELD OF THE INVENTION

The invention is in the field of iontophoresis. In particular, the invention relates to a method for storing current delivery profiles required for delivering drugs in an iontophoretic system.

### BACKGROUND OF THE INVENTION

Iontophoresis is the migration of ions when an electrical current is passed through a solution containing an ionized species, usually the ionic form of a drug or other therapeutic agent (hereinafter referred to as the "drug"). One particularly advantageous application of iontophoresis is the non invasive transdermal delivery of ionized drugs into the skin of a patient using low levels of current. Iontophoretic drug delivery offers an alternative and effective method of drug delivery over other methods such as passive transdermal patches, needle injection, and oral ingestion, and is an especially effective method for children, the bedridden and the elderly. Known advantages of transdermal delivery include avoiding the risks and inconvenience of intravenous delivery. Also, problems associated with oral drug ingestion, such as drug loss caused by digestion and hepatic first pass metabolism are avoided as the gastrointestinal tract and liver (on first pass) are bypassed. Transdermal delivery advantageously provides continuous drug delivery, easy termination and more convenience.

An iontophoresis transdermal drug delivery system usually includes a patch having multiple reservoirs, one of which, called the active reservoir, contains positively- or negatively-charged drug ions, and another one of which, called the return reservoir, contains an electrolytic solution, such as a saline solution. Located within the reservoirs are electrodes for applying current into the patch. The iontophoresis system also includes a controller device, which is electrically and mechanically connected to the patch. The controller usually contains a power source such as a battery, as well as electrical circuitry required for generating and regulating the current applied to the patch electrodes. Preferably, the controller is reused until its battery dies, while the patch is used only once to deliver a full drug dosage and then disposed.

When an iontophoretic delivery device is in operation, an iontophoretic circuit is created. A controller, including a battery, is respectively connected to an anode and cathode in the patch via electrical interconnectors. The anode is arranged in the active reservoir containing a positively- charged drug, while the cathode is arranged in the return reservoir containing the electrolytic (or saline) solution. If the drug is negatively-charged, the anode and cathode arrangement in the reservoirs is reversed, so that when current is applied to the electrodes, drug ions will be repelled from the reservoir of similar polarity. When the patch is placed on the skin of a user and the controller applies current to the patch, the charged drug is forced through the skin of the patient. The return reservoir completes the iontophoretic circuit. For example, if the drug is negatively-charged, a cathode will repel them through the skin.

Notably, the drug flux is a function of the applied current. Accordingly, to increase the amount of drug delivered to the patient, the current applied to the patch is increased, and vice versa. The amount of drug delivered to the patient over time, known as the drug delivery profile, can thus be precisely regulated by regulating the amount of applied current over time, known as the current delivery profile. In general, the current delivery profiles can take on any shape, even including current spikes required for immediate delivery of a large amount of drug, e.g., a bolus dose.

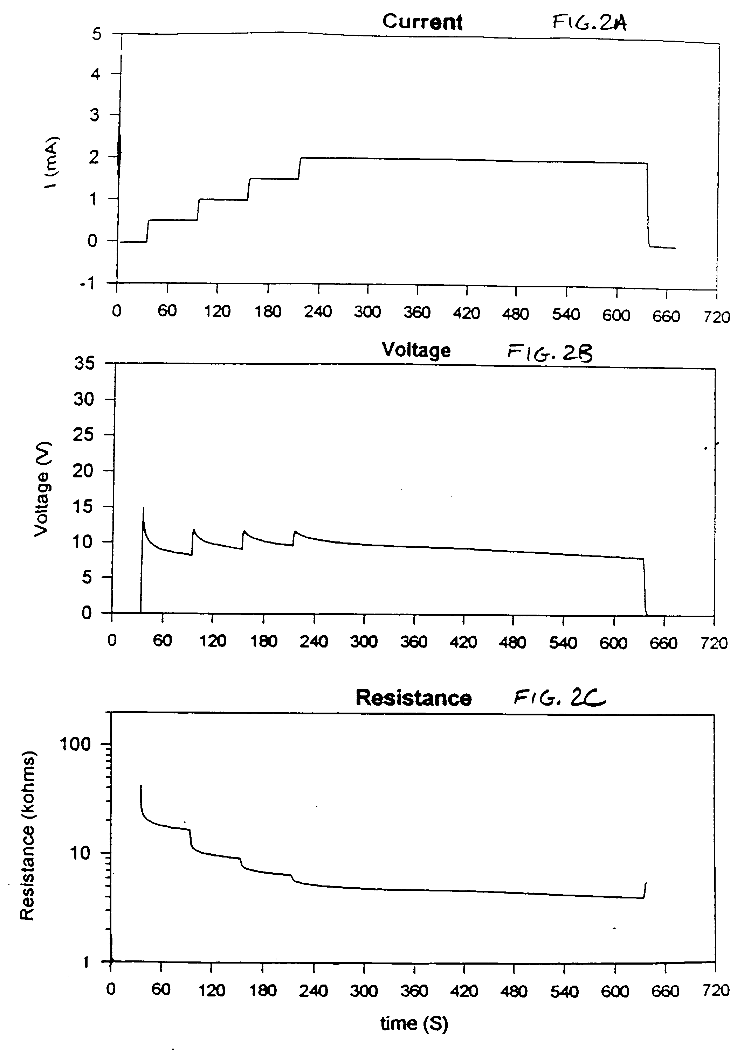

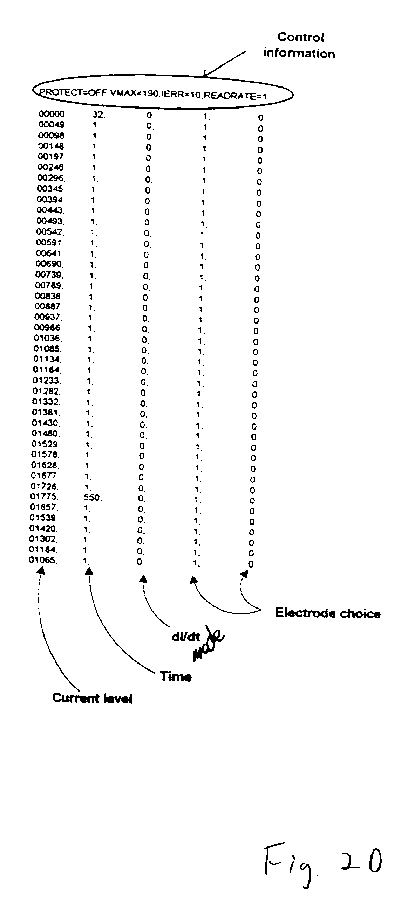

An example of a current delivery profile is shown in Figure 2A. (This current delivery profile, however, is not admitted to be prior art with respect to the present invention by its mention in this Background section.) In this example, the current increases in stepwise fashion from approximately 0 amps to 0.5 milliAmps (mA) at 40 seconds, to 1.0 mA at 90 seconds, to 1.5 mA at 160 seconds, and to 2.0 mA at 220 seconds, and decreases to 0 mA at 630 seconds. The total amount of charge delivered is the area under the curve. Examples of the voltage and resistance measured across the patch corresponding to Figure 2A are shown respectively in Figures 2B and 2C. Spikes in the voltage associated with the steps in applied current are seen in Figure 2B, and may cause unpleasant skin sensations, such as mild stinging or skin irritation.

These unpleasant sensations are primarily caused by the abrupt changes in current (large dI/dt). To reduce these sensations, the current delivery profiles, including the rate of change of current, the maximum current and the duration of current delivery, are carefully designed and implemented for each type of drug delivered, as constrained by the therapeutic drug dosage, the expected range of skin resistance, the maximum output voltage of the controller (the "compliance voltage") and the length of time it takes to deliver the drug.

The controllers may include a microprocessor or state machine to implement the numerous control functions. (These types of controllers, however, are not admitted to be prior art with respect to the present invention by their mention in this Background section.) For example, a microprocessor executes software programs which command the current generation and regulation circuitry to provide the required amount of current over a period of time as dictated by a particular current delivery profile implemented in the software. Under present U.S. and foreign regulations, these software programs must be validated and approved, which is a time-consuming and expensive process.

However, because many different current delivery profiles are necessary depending upon the type of drug delivered, and different controller compliance voltages and electrodes are possible, the software must be continuously changed for each new or changed current delivery profile. In particular, as new drugs appear or drugs change, as new information regarding patient skin characteristics is obtained, as controllers with different compliance voltages or patches with different electrode types are manufactured, the addition of new current delivery profiles or changes in existing current delivery profiles may be required. Thus, the software containing these current delivery profiles must also be changed. The reprogramming of software increases production costs and entails long time delays. Moreover, every time software is changed, the software must be revalidated, further increasing costs and delay.

### SUMMARY OF THE INVENTION

It is thus an object of the present invention to provide a highly flexible method for implementing current delivery profiles in an iontophoresis system without requiring large-scale reprogramming of software, and thus reducing or eliminating revalidation of the device operating system (OS) software.

It is another object of the present invention to provide a method of drug delivery in an iontophoretic delivery system that requires only a single validation of OS software which is designed to implement a plurality of current delivery profiles which are all within a predetermined range.

It is yet another object of the present invention to provide a method of drug delivery in an iontophoretic delivery system that once the software has been validated, the characteristics of the software, primarily the values of the current delivery profiles, are changed by changing only the current delivery profile and not the OS software, thus eliminating the necessity of rewriting and revalidating the OS software, saving both cost and time.

In one aspect of the present invention, an iontophoretic system is provided, including a patch and a controller. The patch includes a plurality of electrodes electrically connectable to the controller and through which current is applied by the controller to the skin. The controller includes at least one current delivery profile, selection circuitry, and circuitry for generating the patch current. The selection circuitry selects a current delivery profile and causes the current generating circuitry to generate and apply current to the patch electrodes in accordance with the selected current delivery profile.

In another aspect of the present invention, the iontophoretic system automatically selects the proper current delivery profile from the memory depending upon the type of patch connected to the controller.

It still another aspect of the present invention, one or more current delivery profiles are received from an external information processing system, such as a computer, by the iontophoretic system.

### BRIEF DESCRIPTION OF THE DRAWINGS

These and other features and advantages of the present invention can be best understood by reference to the detailed description of the preferred embodiments set forth below taken with the drawings, in which:

Figure 1 is a perspective view of an iontophoretic system that includes a patch and a controller.

Figure 2A is an example of a current delivery profile as delivered.

Figures 2B and 2C are respectively measured voltage and resistance curves versus time corresponding to the current delivery profile of Figure 2A.

Figure 2D is an example of a current delivery profile source file.

Figure 3 is an overview of an iontophoretic system and controller circuitry according the first embodiment of the invention.

Figure 4 illustrates using an external host computer to send information to the controller.

Figure 5A is a top view of an interconnection tab of an iontophoretic patch illustrating openings at the end of the tab for identification of the patch.

Figure 5B is a table illustrating the switch state for identifying the type of patch connected to a controller, and the current delivery profile selected when the patch type is identified.

Figure 6 is a flow chart of a method corresponding to the first embodiment of the present invention.

### DETAILED DESCRIPTION OF THE PREFERRED EMBODIMENTS

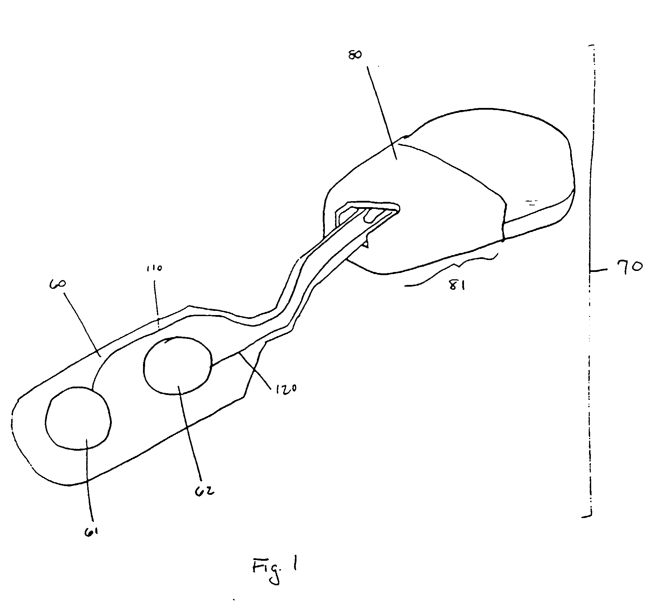

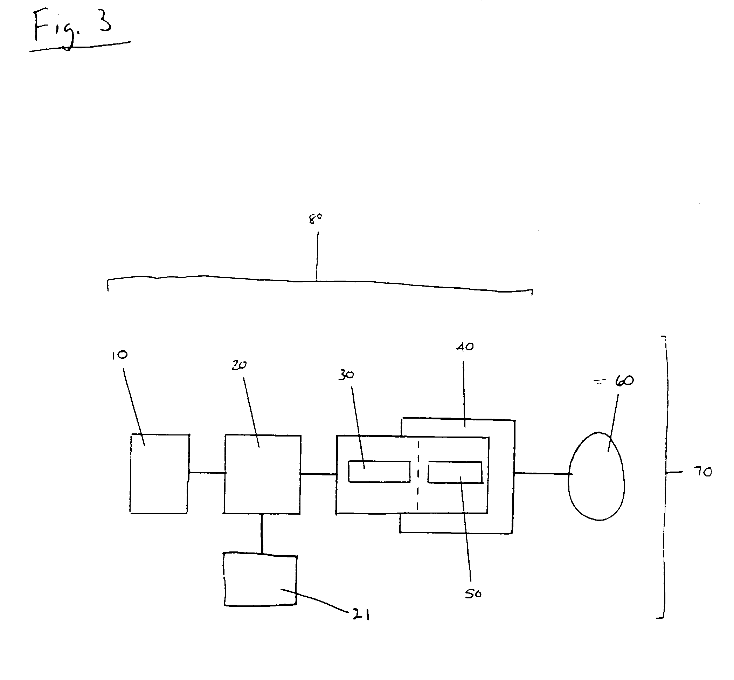

The embodiments of the present invention relate to an iontophoretic system 70 including a patch 60 and a controller 80, as illustrated in Figures 1 and 3. As shown in Figure 1, the controller 80 includes an outer casing 81 which houses the power source and current generation and regulation circuitry required for driving current into the patch 60. The casing of the controller 80 may be made of a plastic, metal or other suitable material for encasing and protecting the current regulating circuitry. The patch electrodes 61 and 62 electrically connect to the current controller 80 via electrical connectors 110 and 120.

As shown in more detail in Figure 3, the controller 80 includes selection/control circuitry 20 (which may comprise, for example, a microprocessor), which upon execution of software, generates commands to control the various functions of the controller, including but not limited to generating and regulating the current applied to the patch as dictated by a predetermined current delivery profile. To accomplish these functions, the selection/control circuitry 20 is connected to the current generation and regulation circuitry, including digital interface and control circuit 30, analog current control circuit 50 and peripheral current control circuitry 40. The peripheral current control circuitry 40 applies the current to the patch 60. The selection/control circuitry 20, digital interface 30, and analog control circuitry 50, together with a module 10 described in detail below, comprise an application specific integrated circuit (ASIC). The peripheral current control circuitry 40 is called "peripheral" because it is outside the ASIC. Of course, it will be appreciated that the current generation and regulation circuitry may take on other configurations, and need not be implemented by an ASIC.

As shown in Figure 3, the selection/control circuitry 20 is connected to an annunciator 21. The annunciator 21 provides status information concerning the iontophoresis system 70. The annunciator can comprise one or more light emitting diodes (LEDs), a liquid crystal display (LCD), a buzzer, a beeper or any other visual and/or audio indicator.

The selection/control circuitry 20 is also connected to at least one module 10 such as random access memory (RAM), a read-only memory (ROM), electronically erasable programmable read-only memory (EEPROM) or flash memory. The software that runs the system (operating system software) may be stored in the same or different module (e.g., memory) as the current delivery profiles. The module 10 may, for example, store each current delivery profile as a set of parameters (rate of change of current (dI/dt), duration of step, electrode pattern, LED pattern, etc. (as described below). Of course, the current delivery profile may increase or decrease as a ramp or other function, e.g., a hyperbolic or a polynomial function. In general, each current delivery profile may be stored as (1) an initial value of the current, usually zero amps, (2) a first current step or dI/dt and associated time, and (3) a second current step and associated time, etc., until the current delivery ends. Alternatively, or in addition to, each current delivery profile may be stored as a discrete set of current (I) values for each point in time, for example, each second.

A hardware description language (HDL) source code module or a hardware implementation of HDL code can also be used to perform the functions of the module 10. In essence, HDLs are used to design application specific integrated circuits (ASIC's). An example of one such programming language is VHDL (Very High Speed Integrated Circuit Hardware Description Language).

The current delivery profiles are created separately of the operating system software of the iontophoretic system 70 and may be transferred into the module 10 at time of manufacture or some later time. These profiles may be in the form of a set of parameters in a table or . as a separate "program" of written instructions that may be interpreted by either the operating system software or the selection/control circuitry 20, once it has been transferred into the iontophoretic system 70.

In addition to current levels, durations, and rate of change, the current delivery profiles may contain control information (e.g., electrode choice and data logging rates) associated with any particular stage of the current delivery and status information. The status information may be used to provide a visual and/or audio indication of the state or status of the iontophoresis system 70. The status information is displayed and/or provided through the annunciator 21. For example, the status information can cause a specific LED pattern to be displayed to indicate that the drug is being delivered properly. Figure 2D is an example of a source code representation of a current delivery profile which includes various parameters as discussed above.

The current delivery profiles may be stored in a look-up table (LUT), wherein each current delivery profile may be associated with a type of drug, type of electrode (or as a combination of drug and electrode as a type of patch), patient characteristics (elderly, children, male, female, skin type), controller type, and the like. The selection/control circuitry 20 "looks up" or reads the correct current delivery profile based on an input into the LUT, for example, the type of patch connected to the controller. Alternatively, the selection/control circuitry may select a predetermined current delivery profile if no input is provided.

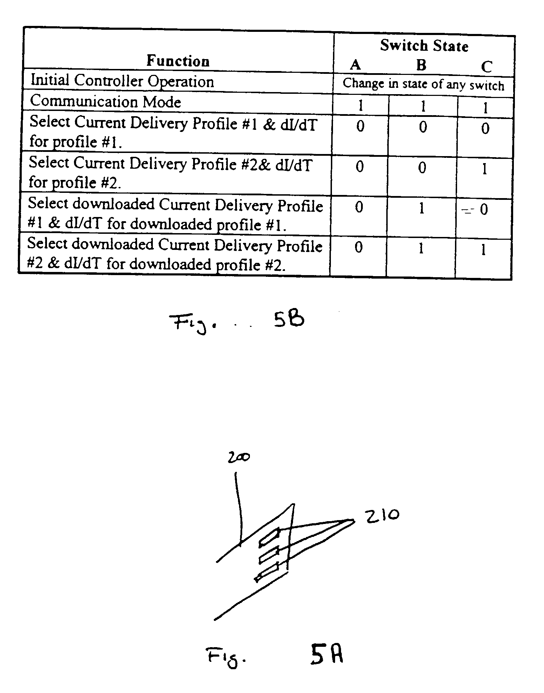

Current delivery profile selection may also be determined automatically by the controller. For example, patch identification openings may exist on the ends of the patch connection tab 200, as shown in Figure 5A. These openings cause the generation of one or more signals upon patch connection, depending on the configuration of the openings, which upon decoding identifies the type of patch connected to the controller. Each type of patch has a different configuration of openings, and will cause a different current delivery profile to be selected. Figure 5B illustrates such an example, showing switch states of a patch having an interconnection tab 200 with up to three identification openings 210 as shown in Figure 5A. When an opening exists, a signal can be generated signifying "on" (1) or "off" (0). In Figure 5B, four different current delivery profiles are identified based on whether openings exist. Using three opening for identifying purposes, a possibility of eight different profiles could be identified.

Of course, it will be appreciated that other forms of patch identification techniques, such as other electromechanical techniques, bar codes or other optical identification codes, or electrical contacts may be used herein. Alternatively, the type of patch, or other criteria for selecting a current delivery profile, may be manually input by a user through a keypad or other known input device integrated with or attached to the controller.

The selection/control circuitry 20 then uses the selected current delivery profile information to generate the appropriately timed commands required to cause the current generation and regulation circuitry to deliver the amount of current dictated by the current delivery profile. In essence, the selection/control circuitry 20 steps through the current delivery profile. At each step, the selection/control circuitry 20 outputs a signal representing a specific current value to a digital to analog (D/A) converter at the specified time (or for a specified duration). The D/A converter is included in the digital interface circuitry 30 (shown in Figure 3). The D/A converter outputs a voltage to a voltage/current conversion circuit (not shown), which generates the required patch current. Feedback from a current sense resistor (not shown) may be used to control this patch current more precisely.

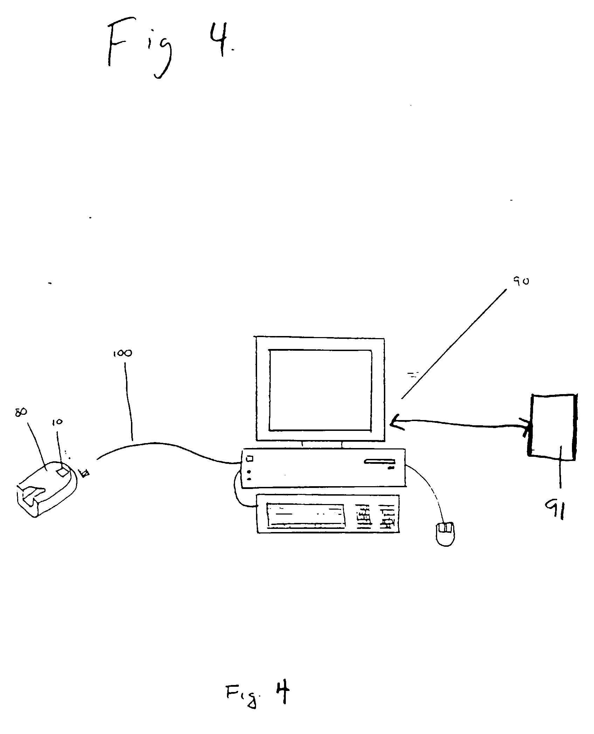

Accordingly, many different current delivery profiles can be stored in the module 10 without changing the OS software. Thus, the OS software only needs to be validated once, presumably for all the anticipated ranges of current delivery profile parameters, but not any specific current delivery profile. Thereafter, any changes in the specific current delivery profile, due to new or changed drugs, electrodes, etc., may be simply downloaded into the memory 10 of controller 80 by an external information processing system via a communication interface (e.g., an external computer 90 and/or an Internet web site 91 as shown in Figure 4). As shown in Figure 4, a host computer 90 and a serial (or parallel) communication line and connector 100 may be used for this purpose. However, it should be understood that other types of communication interfaces may be used, for example, optical, infrared, wireless, Internet web site and radio interfaces.

Alternatively, a new current delivery profile may be stored in a new memory chip, ROM or RAM, which is placed in the controller, or the new information may be "burnt into" an existing EEPROM memory on the controller 80. Since only specific current delivery profile information in the memory has changed, and not the OS software, no reprogramming or revalidation of the OS software is required.

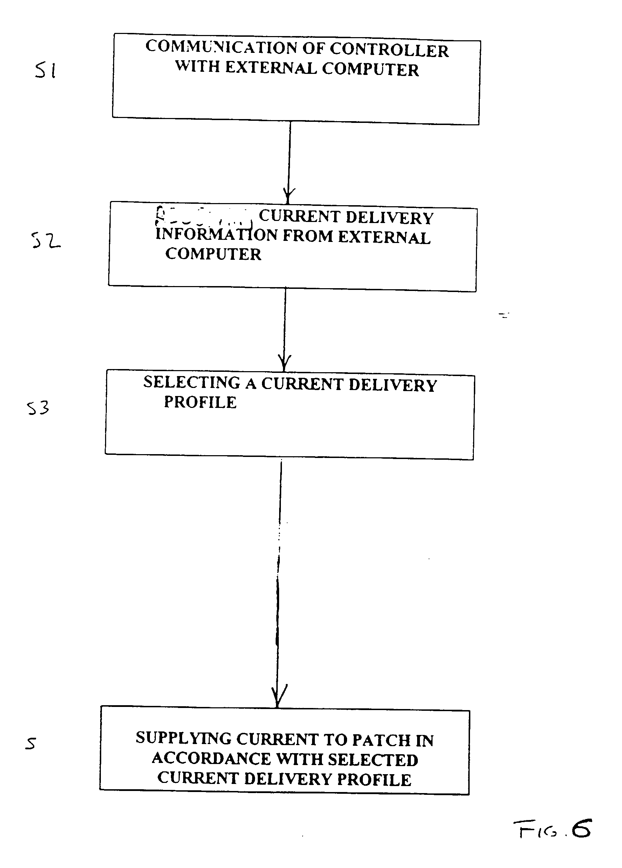

In general, a method of updating the module 10 with new current delivery profiles is shown in Figure 6. In step S1, the external host computer 90 is communicatively connected to the controller 80 and selection circuitry 20 therein via a serial communication line 100. In step S2, updated current delivery profile information is received by the controller 80 from the host computer, replacing the old current delivery profiles. Once received, the host computer and serial communication line are disconnected from the controller, the controller now being ready for iontophoretic drug delivery. Steps S1 and S2 may be performed at a manufacturing facility or may be performed by a health care provider. In step S3, an input can be provided, either automatically or manually as described above, to select a specific current delivery profile. The selection/control circuitry 20 then cycles through the current delivery profile, in step S4, thus causing the generation and application of current to the patch in accordance with the current delivery profile.

Of course, it will be appreciated that the invention may take forms other than those specifically described. For example, a state machine instead of a microprocessor may be used in conjunction with the memory and/or LUT. Accordingly, while the preferred embodiments of the present invention have been described so as to enable one skilled in the art to practice the present invention, it is to be understood that variations and modifications may be employed without departing from the concept and intent of the present invention as defined in the following claims. The preceding description is intended to be exemplary and should not be used to limit the scope of the invention. The scope of the invention should be determined only by reference to the following claims.

## Claims

1. A method for delivering a drug through skin using an iontophoretic system, the iontophoretic system comprising a controller for applying current to a patch, the controller having control circuitry, circuitry for generating the patch current and at least one current delivery profile, the current delivery profile being an element that can be changed independently of the other components of the controller, and the patch having a plurality of electrodes electrically connected to the controller and through which current is applied by the controller to the skin, said method comprising the steps of:
selecting a current delivery profile; and
generating current and applying the same to the electrodes in accordance with the selected current delivery profile.

2. A method according to Claim 1, further comprising the step of inputting an input, and said selecting step selects the current delivery profile in accordance with the input.

3. A method according to Claim 2, wherein in said inputting step, the input is automatically generated based on a decodable key on the patch.

4. A method according to Claim 1, wherein the iontophoretic system further includes a module for storing an operating system which controls the operation of the iontophoretic system.

5. A method according to Claim 1, wherein the control circuitry comprises a microprocessor or a state-machine.

6. A method for delivering a drug through skin using an iontophoretic system, the iontophoretic system comprising control circuitry for applying current to a patch, the control circuitry having circuitry for generating the patch current, and the patch having a plurality of electrodes electrically connectable to the controller and through which current is applied by the controller to the skin, said method comprising the steps of:
communicatively connecting the controller to an external information processing system;
receiving at least one current delivery profile from the external information processing system;
disconnecting the external information processing system and connecting the patch;
selecting a received current delivery profile; and
generating current and applying the same to the electrodes in accordance with the selected current delivery profile.

7. A method according to Claim 6, wherein said selecting step includes receiving an input and selects the current delivery profile in accordance with the input.

8. An iontophoretic system comprising:
a controller;
a patch including a plurality of electrodes electrically connectable to said controller and through which current is applied by said controller to the skin; and
said controller for applying current to said patch, said controller including at least one current delivery profile, control circuitry, and circuitry for generating the patch current, wherein said control circuitry selects a current delivery profile, and causes said current generating circuitry to generate and apply current to said patch electrodes in accordance with the selected current delivery profile.

9. A system according to Claim 8, wherein said controller further includes an input device and said control circuitry selects the current delivery profile based on an input from said input device.

10. A system according to Claim 8, wherein the input is automatically generated based on a decodable key on said patch.
